# EUROPEAN PATENT APPLICATION

(11) **EP 2 903 047 A1**
(43) Date of publication of application: **05.08.2015**
(21) Application number: 14153558.3
(22) Date of filing: 31.01.2014
(51) Int. Cl.: H01L 51/42, C07D 409/14, C07D 495/22

(54) **Hole transporting and light absorbing material for solid state solar cells**

(71) Applicant: Ecole Polytechnique Fédérale de Lausanne (EPFL), 1015 Lausanne (CH)
(72) Inventor: Zakeeruddin, Shaik Mohammad, 1030 Bussigny-Lausanne (CH); Graetzel, Michael, 1025 St-Sulpice (CH); Nazeeruddin, MD. Khaja, 1024 Ecublens (CH); Qin, Peng, 1010 Lausanne (CH); Mishra, Amaresh, 89231 Neu Ulm (DE); Kast, Hannelore, 88453 Erolzheim (DE); Bäuerle, Peter, 89275 Elchingen (DE)
(74) Representative: Ganguillet, Cyril

(57) **Abstract**

The present invention relates to a compound of formula (I) based on compound derived from Acceptor-Donor-Acceptor (A-D-A) oligothiophenes based on heteroacene core and used as hole transporting material in a photovoltaic device, in particular in a solid state solar cell.

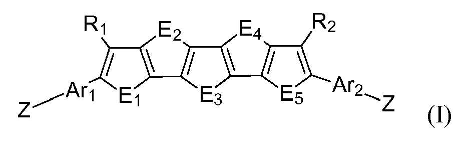

## Description

### Technical Field

The present invention relates to hole transporting and light absorbing material, to hole transporting and light absorbing material for solid state photovoltaic devices, in particular solid state solar cells, and for thin-film photovoltaic devices and organic-inorganic perovskite films or layer photovoltaic devices, to a solid-state heterojunction and flat junction, to a solid state solar cell and to a method for preparing said solid state solar cell.

### Prior Art and the Problem Underlying the Invention

The conversion of solar energy to electrical current using thin film third generation photovoltaics (PV) is being widely explored for the last two decades. The sandwich/monolithic-type PV devices, consisting of a mesoporous photoanode with an organic/inorganic light harvester, redox electrolyte/solid-state hole conductor, and counter electrode, have gained significant interest due to the ease of fabrication, flexibility in the selection of materials and cost effective production.

Recently, organic-inorganic hybrid systems based on organo-metal halide perovskites with general formula (RNH₃)BX₃ (R = alkyl, B = Pb, X = I, Br or Cl) have drawn considerable success as light harvesters in solid-state heterojunction solar cells. Their opto-electronic properties can be easily tuned by changing the alkyl group or by variation of the halogen atoms. These perovskite nanoparticles have direct band gap, large absorption coefficient and high charge carrier mobility. Unpublished European patent application EP 12179323.6 disclosed a solid-state solar cell comprising one or more organic-inorganic perovskite layers and showing remarkable conversion efficiencies even though in absence of an organic hole transporting material.

In 2009, perovskites have been employed as sensitizer in photoelectrochemical cell using liquid electrolyte with power conversion efficiencies (PCE) from 3.8-6.5%. However, the device performance dramatically reduces due to dissolution of the perovskite in the electrolyte. This problem was solved by using 2,2',7,7'-tetrakis(N,N-di-p-methoxyphenyl-amine)9,9'-spirobifluorene (Spiro-MeOTAD) as a solid state hole transport material (HTM), generating a PCE of 9.7%.

The PCE of such solar cells was increased by improving the method of producing the perovskite layer in situ onto the mesoporous TiO₂ films. From experience, the morphology of the perovskite crystals formed during solution processing cannot be well controlled and is one of the reasons for the poor reproducibility of photovoltaic cell performance. Unpublished European patent application EP 13166720.6 disclosed an efficient and reproducible method for the application of the light harvester layer of perovskite on the nanoporous layer of the current collector. The two precursors of the organic-inorganic perovskite being in solution are separately applied on the nanoporous layer of the current collector in a two-step deposition, namely a first step for forming a film on the nanoporous layer with the first precursor and a second step for applying a film of the second precursor, to obtain a layer comprising the organic-inorganic perovskite pigment. Recently, solid-state solar cells being prepared according to this method and comprising the hybrid organic-inorganic perovskite CH₃NH₃PbX₃, X being Cl⁻, Br⁻ or I⁻, in combination with spiro-MeOTAD (2,2',7,7'-tetrakis(N,N-di-p-methoxyphenylamine) -9,9'-spirobifluorene) as organic hole transporting material (HTM) achieved power conversion efficiency (PCE) of 15% under full illumination.

However, the electron-hole diffusion lengths in a solution-processed CH₃NH₃PbI₃ perovskite layer is reported to be about 100 nm, indicating that the photogenerated charges cannot be efficiently extracted in thicker films.

In perovskite-based devices, the efficient HTMs used are mainly limited to wide band gap spiro-MeOTAD or poly(triarylamine) (PTAA), which show good hole mobilities, but almost no light harvesting ability in the visible and near-IR region. Some low band gap donor (D)-acceptor (A) polymers with highest occupied molecular orbital (HOMO) in the range from 5.2-5.4 eV have been used as HTM in CH₃NH₃PbI₃-based solar cells, exhibiting PCEs in the range from 4.2% to 6.7%. In these devices the polymers only contribute as HTM and perovskite plays the role of photon absorption.

Further, the use of spiro-MeOTAD as hole transporting material may trigger instability in such solid-state solar cells. Because Spiro-MeOTAD has two oxidation potentials being very close, this hole transporting material in the oxidized form is able to form a dication, which in turn can dismutate and might cause device instability.

The present invention addresses the disadvantage of organic hole transporting material, which provides instability to the device, when said hole transporter material is in oxidized form, as it is the case for spiro-MeOTAD.

The present invention also pursues to provide new hole transporting material, which provides a higher PCE to the solid-state photovoltaic devices comprising perovskite as sensitizer or light absorbing material.

The present invention addresses the disadvantage of the perovskite pigment, which cannot absorb the complete incident light, in particular in the visible and the near-infrared parts of the light spectrum and to absorb the remnant light passing through the layer comprising the perovskite pigment to increase the photoconversion and photocurrent generation of the whole device and therefore the efficiency and the performance of the photovoltaic device.

The invention also addresses the low ability of light harvesting of CH₃NH₃PbI₃-based devices.

The invention pursues to provide an efficient solar cell, which can be rapidly prepared in an efficient way, using readily available or low cost materials such as conductive material, using a short manufacturing procedure based on industrially known manufacturing steps, keeping the material costs and the material impact on the environment very low.

The present invention addresses the problems depicted above.

### Summary of the Invention

Remarkably, in some aspects, the present inventors have found that a compound derived from heteroacene based oligothiophene operates as a hole transporting material and as a light harvester or a light absorbing material and improves the PCE of solid photovoltaic devices comprising perovskite pigment as sensitizer. Said compound may absorb light to the near IR regions of the light spectrum, namely from 600 nm to 800 nm. In a solid state photovoltaic device, said compound also absorbs the light in the range of the light spectrum being not absorbed by the sensitizer layer and in particular a sensitizer layer comprising organic-inorganic perovskite pigment.

The specific configuration of the structure of the compound of the invention being Acceptor-Donor-Acceptor (A-D-A) oligothiophenes based on a heteroacene core provides a broad absorption and a fine tuning of the frontier orbital energies of the free charges extracted from the sensitizer layer, in particular from the perovskite layer.

These HTMs contribute to both the effective charge extraction, and photocurrent enhancement in the solid photovoltaic device.

Although their large size, said compounds are good soluble in organic solvents, which greatly facilitates their purification and processing and their application or deposition on the sensitizer layer in the solid photovoltaic device.

In an aspect, the present invention provides compound of formula (I): 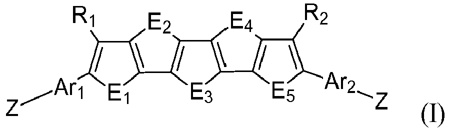 wherein
- E₁, E₂, E₃, E₄ and E₅ are independently selected from the group consisting of O, S, Se, CR₂, SiR₂ or NR, wherein R is selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted;
- R₁ and R₂ are independently selected from H, C1-C20 alkyl, C1-C20 alkoxy, C2-C20 alkenyl, C4-C20 aryl or C1-C20 fluoroalkyl group, wherein said alkyl, alkenyl, alkynyl, alkoxy, and fluoroalkyl, if they comprise 3 or more carbons may be linear, branched or cyclic;
- Ar₁ and Ar₂ are identical or different moieties selected from heteroaromatic ring system having one or more aromatic rings comprising 5 to 40 ring atoms and one or more heteroatoms independently selected from N, S, Se, CR₂, SiR₂, O or NR, wherein R is defined as above, wherein said aromatic ring may further substituted by one or more moieties independently selected from H, keto (=O) group, fluoro group, C1-C20 alkyl, C1-C20 alkoxy, C2-C20 alkenyl and C2-C20 alkynyl group;
- Z is, on each occurrence, a moiety identically or differently selected from C2-C20 alkenyl, C2-C20 alkynyl, C2-C20 cyanoalkenyl, C2-C20 dicyanoalkenyl, C2-C20 cyanoalkenyl carboxylic ester derivative, C2-C20 alkenyl dicarboxylic ester derivative or C4-C20 heteroaryl, C4-C20 alkenylheteroaryl, wherein one or more heteroatoms are independently selected from O, S or NR, R being defined as above, wherein said alkenyl, cyanoalkenyl, dicyanoalkenyl, cyanoalkenyl carboxylic ester derivative, alkenyl dicarboxylic ester derivative, heteroaryl and alkenyl heteroaryl may be further substituted by one or more moieties independently selected from H, C4-C20 aryl, C1-C20 alkyl, C1-C20 alkoxy group, keto (=O) group, cyano group, C2-C20 dicyanoalkenyl, C2-C20 cyanoalkenyl, carboxylic ester derivative group or dicarboxylic ester derivative group.

In a further aspect, the invention provides a photovoltaic solid state device comprising a compound of the invention of formula (I).

According to an embodiment, the invention provides more specifically a photovoltaic solid state device comprising a compound of the invention of formula (I) and further comprising an organic-inorganic perovskite as sensitizer and being under the form of a layer.

Further aspects, further embodiments and preferred embodiments of the invention are detailed herein below and in the appended claims. Further features and advantages of the invention will become apparent to the skilled person from the description of the preferred embodiments given below.

### Brief Description of the Drawings

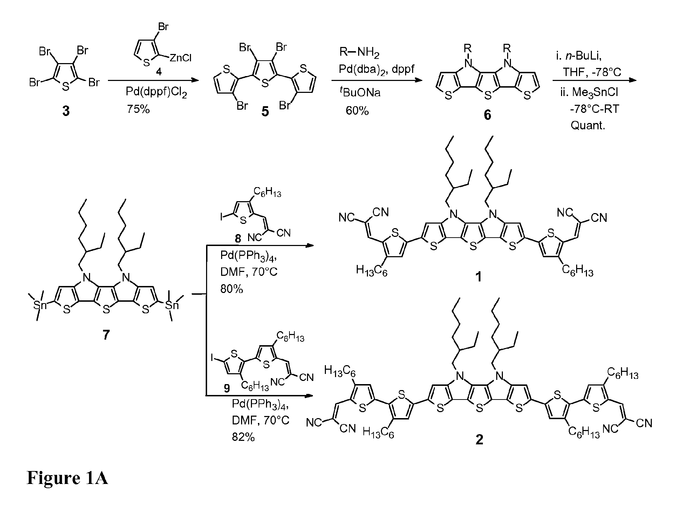

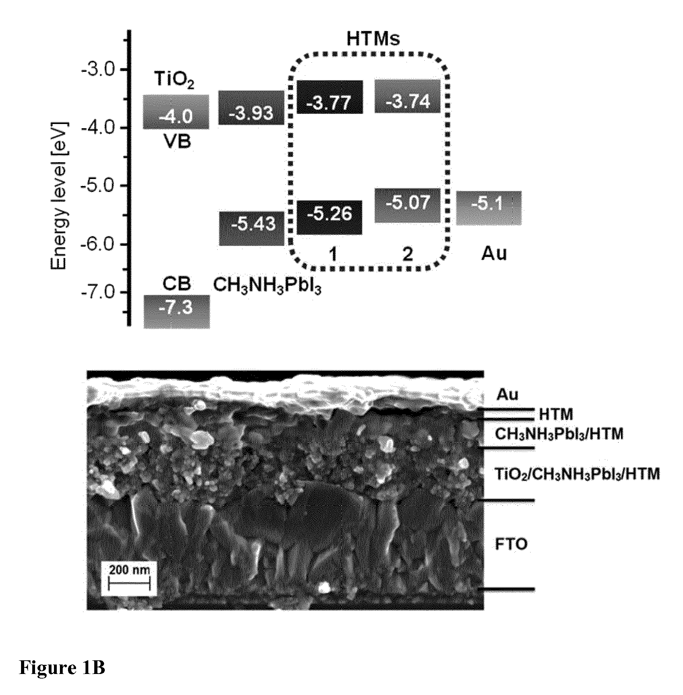

**Figure 1A** shows the schematic representation of the synthesis of compound of formula (35), namely compound 1, and of compound of formula (36), namely compound 2. Figure **1B** shows, on top, the Energy level diagram of said compounds 1 and 2 used as hole transporting material (HTM) in the TiO₂/CH₃NH₃PbI₃/HTM/Au heterojunction solar cell, and on bottom, a cross-sectional SEM image of a photovoltaic solid state device of the invention having either compound 1 or compound 2 as HTM.

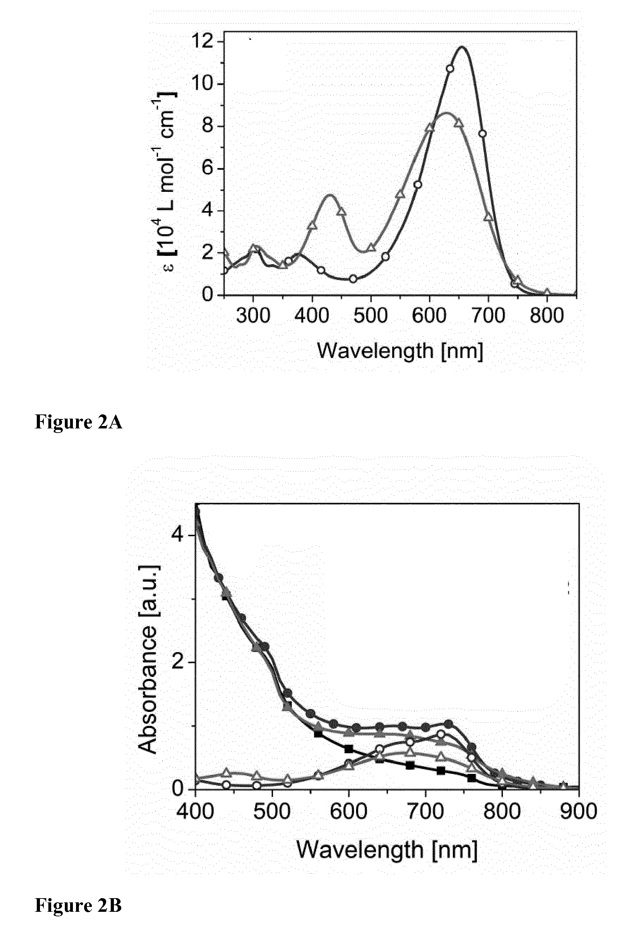

**Figure 2A** shows the UV-visible light absorption spectra of compound of formula (35) (compound 1: open circles) and of compound of formula (36) (compound 2: open triangles).
**Figure 2B** shows the UV-visible light absorption spectra of TiO₂/CH₃NH₃PbI₃ film not coated by HTM: full squares; TiO₂/CH₃NH₃PbI₃ film coated by compound of formula (35) (compound 1): full circles, or compound formula (36) (compound 2): full triangles; mesoporous TiO₂ coated by compound of formula (35) (compound 1): open circles, or by compound of formula (36) (compound 2): open triangles.

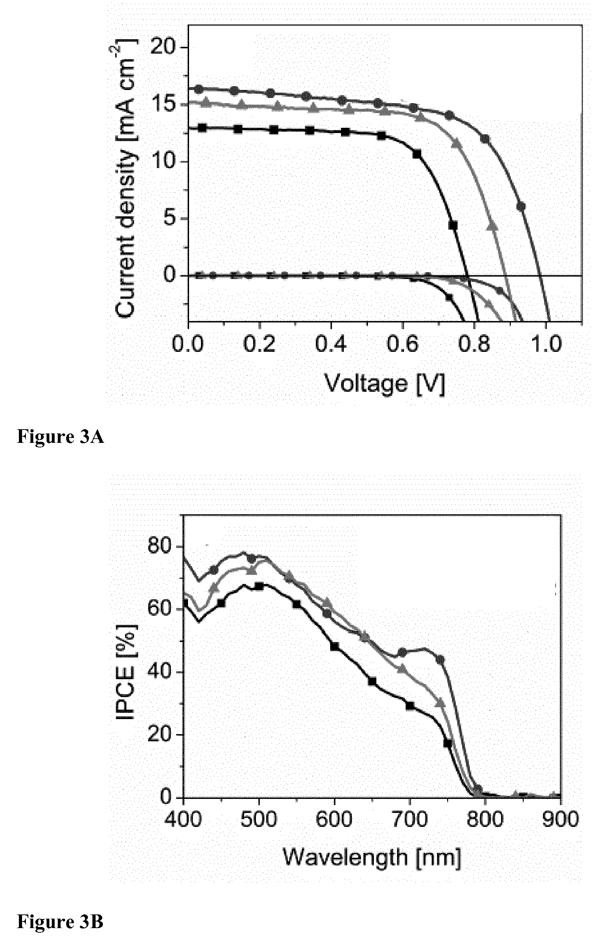

**Figure 3A** shows Current-Voltage (*J*-V) characteristics of a photovoltaic device/heterojunction solar cell comprising perovskite (CH₃NH₃PbI₃) uncoated by HTM: full squares; comprising perovskite coated by compound of formula (35): full circles or by compound of formula (36): full triangles, measured under standard global AM 1.5 sunlight
**Figure 3B** shows Incident Photon to Converted Electron (IPCE) spectra of a photovoltaic device/heterojunction solar cell comprising perovskite (CH₃NH₃PbI₃) uncoated by HTM: full squares; comprising perovskite coated by compound of formula (35): full circles or by compound of formula (36): full triangles, measured under standard global AM 1.5 sunlight.

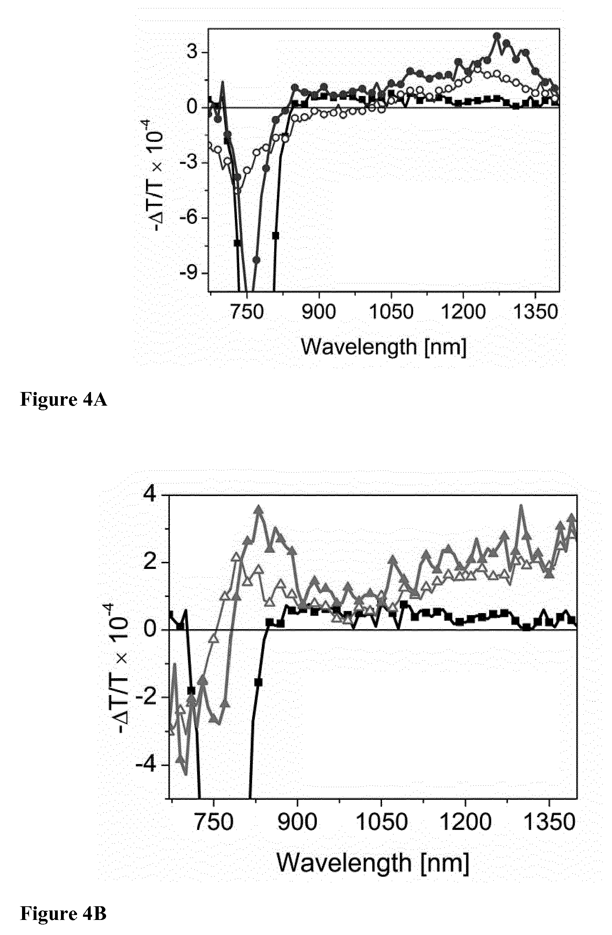

**Figure 4A** shows the photoinduced absorption (PIA) spectra of a photovoltaic device/solar cell comprising a mesoporous TiO₂ films coated by perovskite (CH₃NH₃PbI₃): full squares, coated by compound of formula (35) as HTM: open circles, and coated by perovskite and compound of formula (35): full circles measured at a wavelength of 642nm. Figure 4B shows the photoinduced absorption (PIA) spectra of a photovoltaic device/solar cell comprising a mesoporous TiO₂ films coated by perovskite (CH₃NH₃PbI₃): full squares, coated by compound of formula (36) as HTM: open triangles, and coated by perovskite and compound of formula (36): full triangles measured at a wavelength of 642nm.

### Detailed Description of the Preferred Embodiments

The present invention concerns a compound based Acceptor-Donor-Acceptor (A-D-A) oligothiophenes based on heteroacene core.

In particular the present invention concerns a compound of formula (I). 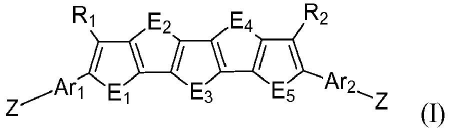 wherein
- E₁, E₂, E₃, E₄ and E₅ are independently selected from the group consisting of O, S, Se, CR₂, SiR₂ or NR, wherein R is selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted;
- R₁ and R₂ are independently selected from H, C1-C20 alkyl, C1-C20 alkoxy, C2-C20 alkenyl, C2-C20 alkynyl, C4-C20 aryl or C1-C20 fluoroalkyl group, wherein said alkyl, alkenyl, alkynyl, alkoxy, and fluoroalkyl, if they comprise 3 or more carbons may be linear, branched or cyclic;
- Ar₁ and Ar₂ are identical or different moieties selected from heteroaromatic ring system having one or more aromatic rings comprising 5 to 40 ring atoms and one or more heteroatoms independently selected from N, S, Se, CR₂, SiR₂, O or NR, wherein R is defined as above, wherein said aromatic ring may further substituted by one or more moieties independently selected from H, keto (=O) group, fluoro group, C1-C20 alkyl, C1-C20 alkoxy, C2-C20 alkenyl and C2-C20 alkynyl group;
- Z is, on each occurrence, a moiety identically or differently selected from C2-C20 alkenyl, C2-C20 alkynyl, C2-C20 cyanoalkenyl, C2-C20 dicyanoalkenyl, C2-C20 cyanoalkenyl carboxylic ester derivative, C2-C20 alkenyl dicarboxylic ester derivative or C4-C20 heteroaryl, C4-C20 alkenylheteroaryl, wherein one or more heteroatoms are independently selected from O, S or NR, R being defined as above, wherein said alkenyl, cyanoalkenyl, dicyanoalkenyl, cyanoalkenyl carboxylic ester derivative, alkenyl dicarboxylic ester derivative, heteroaryl and alkenyl heteroaryl may be further substituted by one or more moieties independently selected from H, C4-C20 aryl, C1-C20 alkyl, C1-C20 alkoxy group, keto (=O) group, cyano group, C2-C20 dicyanoalkenyl, C2-C20 cyanoalkenyl, carboxylic ester derivative group or dicarboxylic ester derivative group.

Di- and carboxylic ester derivatives may be selected from hydroxyl acid, alkoxy acid, oxo acid, peroxy acid, salt, ester, acyl halide or acid anhydrides.

In an embodiment of the compound of formula (I), E₂ and E₄ are identical moieties selected from the group consisting of O, S, Se, CR₂, SiR₂ and NR and E₁, E₃ and E₅ are identical moieties selected from the group consisting of O, S, Se, CR₂, SiR₂ and NR, said R being selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted; and said E₂ and E₄ identical moieties are different to said E₁, E₃ and E₅ identical selected moieties.

In another embodiment, when E₂ and E₄ of the compound of formula (I) are identical moieties and E₁, E₃ and E₅ of the compound of formula (I) are identical moieties, said compound is a compound of formula (II) 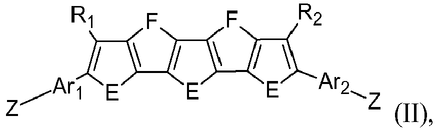 wherein F representing E₂ and E₄ are moieties identically selected from the group consisting of O, S, Se, CR₂, SiR₂ and NR and E₁, E₃ and E₅ are identical moieties selected from the group consisting of O, S, Se, CR₂, SiR₂ and NR, said R being selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted. In the compound of formula (II), F moieties are different to E moieties.

In another embodiment of the compound of formula (I), E₁, E₂, E₄ and E₅ are identical moieties selected from the group consisting of O, S, Se, CR₂, SiR₂ and NR and E₃ is a moiety selected from the group consisting of O, S, Se, CR₂, SiR₂ and NR, wherein R is selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted; and said E₁, E₂, E₄ and E₅ identical moieties are different to said E₃ selected moiety.

In another embodiment, when E₁, E₂, E₄ and E₅ are identical moieties, the compound of the invention is of formula (III) 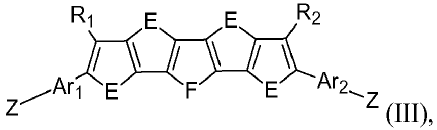 wherein E representing E₁, E₂, E₄ and E₅ are moieties selected from the group consisting of O, S, Se, CR₂, SiR₂ and NR and F representing E₃ is a moiety selected from the group consisting of O, S, Se, CR₂, SiR₂ and NR, wherein R is selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted. In the compound of formula (III), F moiety is different to E moieties.

In an embodiment of the compounds of invention of formula (I) and/or of formula (II) and/or of formula (III), Ar₁ and Ar₂ are independently selected from a moiety according to any one of the formulae (1) to (19) 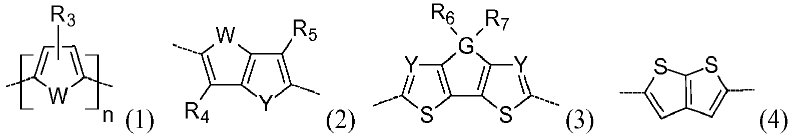 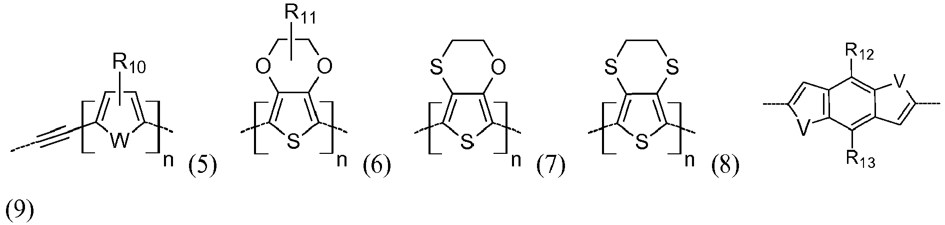 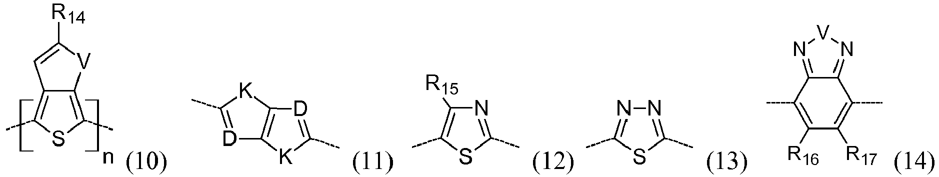 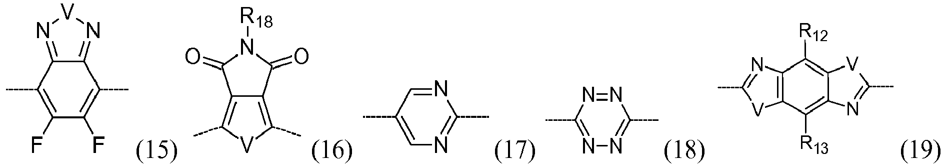 wherein
- W is independently selected from O, S, Se, or NR and Y, V, K and D are independently selected from C, N, S, Se, O, CR₂, SiR₂ or NR, said R being selected from C1-C20 alkyl, C2-C20 alkenyl, C4-C20 aryl and C1-C20 fluoroalkyl group, wherein said alkyl, alkenyl, alkynyl and fluoroalkyl, if they comprise 3 or more carbons may be linear, branched or cyclic and wherein aryl group may be substituted;
- G is selected from C or Si;
- n is an integer from 1 to 10;
- R₃-R₁₈ are independently selected from from H, C1-C20 alkyl, C1-C20 alkoxy, C2-C20 alkenyl.

In a preferred embodiment, in any one of moieties of formulae (1) to (19), V is independently selected from O, S or Se atoms and K and D are independently selected form C, O or N atoms; W, Y, K and G are defined as above. Preferably K is a different moiety to D and W is a different moiety to Y. Preferably K of formula (6) is an identical moiety on each occurrence. Preferably D of formula (6) is an identical moiety on each occurrence. Preferably V is an identical moiety on each occurrence.

In preferred embodiment, Ar1 and Ar2 are selected from a moiety according to any one of the formulae (1) to (3), (6) to (8), (11) and (12). Most preferably, Ar1 and Ar2 are selected from a moiety according to any one of the formulae (1) to (3), (11) and (12).

According to a further embodiment, Ar₁ and Ar₂ are different or identical substituents. Ar₁ and Ar₂ are preferably identical substituents.

In an embodiment of the compounds of invention of formula (I) and/or of formula (II) and/or of formula (III), Z is selected from a moiety according to any one of the formulae (20) to (34) and (58) to (66) 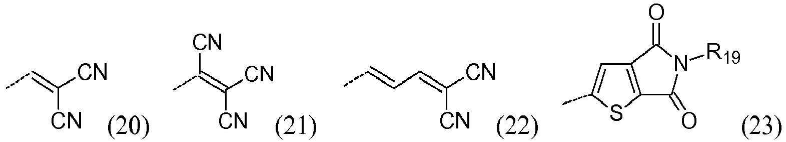 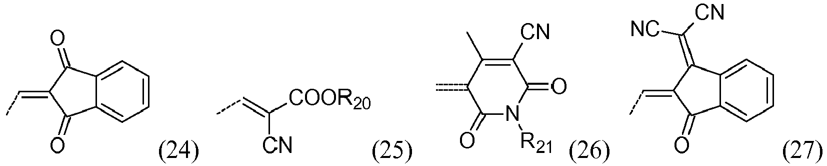 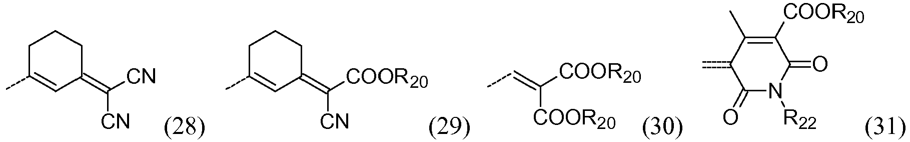 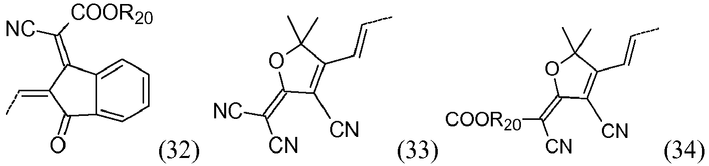 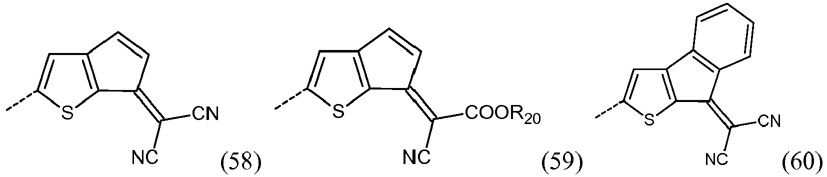 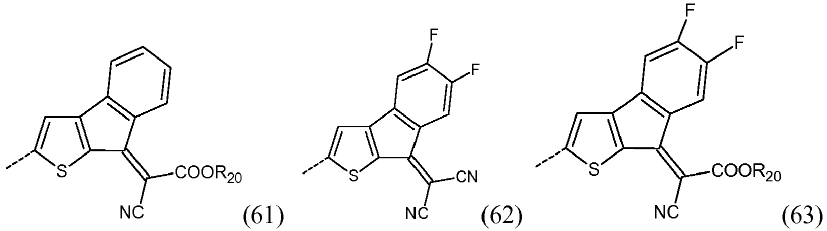 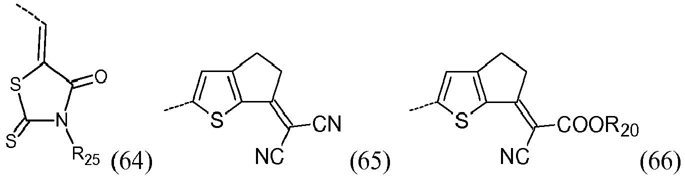 wherein
- COOR₂₀ is a carboxylic ester derivative, wherein R₂₀ is selected from C1-C20 alkyl group;
- R₁₉, R₂₁, R₂₂ and R₂₅ are independently selected from H, C1-C20 alkyl, C1-C20 alkoxy, C2-C20 alkenyl, C2-C20 alkynyl, C1-C20 fluoroalkyl group and C4-C20 aryl, wherein said alkyl, alkenyl, alkoxy, alkynyl and fluoroalkyl, if they comprise 3 or more carbons may be linear, branched or cyclic and wherein aryl group may be substituted.

In a preferred embodiment, Z is selected from a moiety according to any one of the formulae (20) to (29), (32) to (34), (58), (60), (62) (64) and (65). Most preferably, Z is selected from a moiety according to any one of the formulae (20) to (24), (27), (58), (60), (62) (64) and (65).

In a further embodiment, Z is, on each occurrence, a substituent different or identical to each other. Preferably, Z is, on each occurrence, identical substituent.

According to an embodiment of the compounds of the invention of formula (I), of formula (II) and/or of formula (III), R₁ and R₂ are identical substituents. In another embodiment, R₁ and R₂ are identical and selected from H, C1-C20 alkyl, C1-C20 alkoxy, C2-C20 alkenyl, C2-C20 alkynyl, C4-C20 aryl or C1-C20 fluoroalkyl group, wherein said alkyl, alkenyl, alkynyl, alkoxy, and fluoroalkyl, if they comprise 3 or more carbons may be linear, branched or cyclic, if they comprise 3 or more carbons may be linear, branched or cyclic, preferably branched.

Preferably alkyl, alkoxy, alkenyl, alkynyl, cyanoalkenyl, dicyanoalkenyl, fluoroalkyl group, cyanoalkenyl carboxylic ester derivative, alkenyl dicarboxylic ester derivative, aryl heteroaryl, alkenylheteroaryl of substituents R, R₁ to R₁₉ and R₂₁ and R₂₅ are selected from hydrocarbon containing from 1 to 16 carbons, 1 to 12 carbons, 1 to 8 carbons, 4 to 16 carbons, and 4 to 12 carbons and may contain 0-10 heteroatoms being selected form O, N, S, Se, Si, CR₂, SiR₂ or NR, R being defined as above, and wherein said alkyl, alkoxy, alkenyl, alkynyl, fluoroalkyl, cyanoalkenyl carboxylic acid derivative and alkenyl dicarboxylic acid derivative, if they comprise 3 or more carbons may be linear, branched or cyclic, preferably branched. Substituents R, R₁ to R₁₉ and R₂₁ and R₂₅ substituting the same moiety of same formula may be identical to the other substituent substituting the same moiety of same formula or different. For example, R₆ and R₇ substituting the moiety of formula (3) may be identical or different. Preferably said substituents R, R₁ to R₁₉ and R₂₁ and R₂₅ are identical to the other substituent substituting the same moiety of same formula.

The dotted line in the substituents or moieties of formulae (1) to (34) represents the bond by which said substituent is connected to either the heteroacene core or to the previous or the following moieties.

According to a further embodiment, the compound of formula (I) and/or of formula (II) and/or of formula (III) is selected from a compound according to any one of formulae (35) and (36):

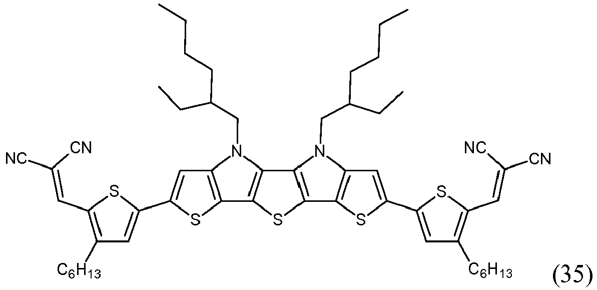

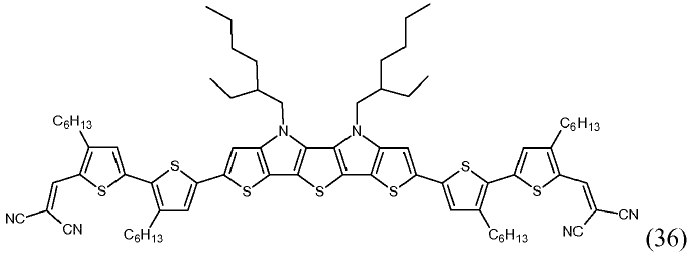

The invention also provides in another aspect a photovoltaic solid state device comprising a compound of formula (I). Said device may comprise a compound of formula (II) and/or of formula (III).

The photovoltaic solid state device is selected from a solar cell, a heterojunction, an optoelectronic device, a light emitting device. Preferably said photovoltaic solid state is a solar cell, preferably a solid state solar cell. Preferably the heterojunction is a solid heterojunction.

According to an embodiment, the photovoltaic solid state device of the invention comprises a conducting support layer, a surface-increasing scaffold structure, a sensitizer or sensitizer layer, a hole transporting layer and a counter electrode and/or metal layer.

In an embodiment, the hole transporting layer of the photovoltaic state device is made of hole transporting material and comprising a compound of formula (I). Said hole transporting layer may comprise a compound of formula (I) and/or of formula (II) and/or formula (III).

According to an embodiment, the conducting support layer, the scaffold structure, the sensitizer layer and the counter electrode are present in this order from one side to the other of the solar cell of the invention. A protective layer may or may not be present, for example at appropriate positions between the above layers, as disclosed elsewhere in this specification.

According to another embodiment, the photovoltaic device comprises a hole collector layer, a conductive layer, an electron blocking layer, a sensitizer layer and a current collector layer, wherein the hole collector layer is coated by the conductive layer; wherein the electron blocking layer is between the conductive layer and the sensitizer layer, which is in contact with the current collector layer being a metal or a conductor.

According to a further embodiment, the conductive material is selected from one or more conductive polymers or one or more hole transporting materials, which may be selected from poly(3,4-ethylenedioxythiophene):poly(styrenesulfonate) (PEDOT:PSS), poly(3,4-ethylenedioxythiophene):poly(styrenesulfonate):grapheme nanocomposite (PEDOT:PSS:graphene), poly(N-vinylcarbazole) (PVK) and sulfonated poly(diphenylamine) (SPDPA), preferably from PEDOT:PSS, PEDOT:PSS:graphene and PVK, more preferably from PEDOT:PSS. Conductive polymers may also be selected from polymers comprising polyaniline, polypyrrole, polythiophene, polybenzene, polyethylenedioxythiophene, polypropylenedioxy-thiophene, polyacetylene, and combinations of two or more of the aforementioned, for example. The conductive polymer of the invention is preferably selected from the above polymer in a watery dispersion.

For the purpose of the present specification, the expression "in electric contact with" means that electrons or holes can get from one layer to the other layer with which it is in electric contact, at least in one direction. In particular, considering the electron flow in the operating device exposed to electromagnetic radiation, layers through which electrons and/or holes are flowing are considered to be in electric contact. The expression "in electric contact with" does not necessarily mean, and preferably does not mean, that electrons and/or holes can freely move in any direction between the layers.

The conducting support layer is preferably substantially transparent. "Transparent" means transparent to at least a part, preferably a major part of the visible light. Preferably, the conducting support layer is substantially transparent to all wavelengths or types of visible light. Furthermore, the conducting support layer may be transparent to non-visible light, such as UV and IR radiation, for example.

According to an embodiment, the conducting support layer provides the support layer of the solar cell of the invention. Preferably, the solar cell is built on said support layer. According to another embodiment, the support of the solar cell is provided on the side of the counter electrode. In this case, the conductive support layer does not necessarily provide the support of the device, but may simply be or comprise a current collector, for example a metal foil.

The conducting support layer preferably functions and/or comprises a current collector, collecting the current obtained from the solar cell. The conducting support layer may comprise a material selected from indium doped tin oxide (ITO), fluorine doped tinoxide (FTO), ZnO-Ga₂O₃, ZnO-Al₂O₃, tin-oxide, antimony doped tin oxide (ATO), SrGeO₃ and zinc oxide, preferably coated on a transparent substrate, such as plastic or glass. In this case, the plastic or glass provides the support structure of the layer and the cited conducting material provides the conductivity. Such support layers are generally known as conductive glass and conductive plastic, respectively, which are thus preferred conducting support layers in accordance with the invention. According to an embodiment, the conducting support layer comprises a conducting transparent layer, which may be selected from conducting glass and from conducting plastic.

According to an embodiment of the invention, a surface-increasing scaffold structure is provided on said conducting support structure or on a protective layer that may be provided on said scaffold structure.

According to an embodiment of the solar cell and the heterojunction of the invention, the surface-increasing scaffold structure is nanostructured and/or nanoporous. The scaffold structure is thus preferably structured on a nanoscale. The structures of said scaffold structure increase the effective surface compared to the surface of the conductive support.

According to an embodiment, said scaffold structure is made from and/or comprises a metal oxide. For example, the material of the scaffold structure is selected from semiconducting materials, such as Si, TiO₂, ZrO₂, Al₂O₃, SnO₂, Fe₂O₃, ZnO, WO₃, Nb₂O₅, CdS, ZnS, PbS, Bi₂S₃, CdSe, CdTe, SrTiO₃, GaP, InP, GaAs, CuInS₂, CuInSe₂, and combinations thereof, for example. Preferred semiconductor materials are Si, TiO₂, ZrO₂, Al₂O₃, SnO₂, ZnO, WO₃, Nb₂O₅ and SrTiO₃, for example. According to an embodiment, the surface-increasing scaffold structure is nanostructured and/or nanoporous.

The invention does not intend to exclude the possibility that there are one or more intermediate layers between the scaffold structure and the conductive support. Such intermediate layers, if present, would preferably be conducting and/or semiconducting.

According to an embodiment, the sensitizer layer of the photovoltaic device comprising at least one pigment being selecting from organic, inorganic, organometallic and organic-inorganic pigments or a combination thereof. The sensitizer is preferably a light absorbing compound or material. Preferably, the sensitizer is a pigment, and most preferably the sensitizer is an organic-inorganic pigments.

The sensitizer layer may comprise one or more pigments of the group consisting of organometallic sensitizing compounds (pthalocyanine derived compounds, porphyrine derived compounds), metal free organic sensitizing compounds (diketopyrrolopyrrole (DPP) based sensitizer), inorganic sensitizing compounds such as quantum dots, Sb₂S₃ (Antimonysulfide, for example in the form of thin films), aggregates of organic pigments, nanocomposites, in particular organic-inorganic perovskites, and combinations of the aforementioned. For the purpose of the invention, it is in principle possible to use any type of dyes or sensitizer, including combinations of different types of dyes or different dyes of the same type.

According to one embodiment, the photovoltaic device of the invention comprising a compound of formula (I) further comprises an organic-inorganic perovskite as sensitizer, said sensitizer being under the form of layer. In Said photovoltaic device, the compound of formula (I) may be a compound of formula (II) or formula (III) or a combination thereof.

According to an embodiment, the sensitizer layer of the photovoltaic device of the invention is coated by a layer comprising the compound of formula (I) and/or of formula (II) and/or of formula (III). Preferably said sensitizer layer comprises an organic-inorganic perovskite as sensitizer.

According to an embodiment, the sensitizer or the sensitizer layer comprises, consists of or is made of an organic-inorganic perovskite. Said organic-inorganic perovskite is provided under a film of one perovskite pigment or mixed perovskite pigments or perovskite pigments mixed with further dyes or sensitizers.

According to a further embodiment, the sensitizer layer comprises a further pigment in addition to the organic-inorganic perovskite pigment, said further pigment selected from organic pigment, organometallic pigment or inorganic pigment.

Organometallic sensitizers are disclosed, for example, in EP0613466, EP0758337, EP 0983282, EP 1622178, WO 2006/038823, WO2009/107100, WO2010/055471 and WO2011/039715. Exemplary organic dyes are those disclosed in WO2009/098643, EP1990373, WO 2007/100033 for example. An organic dye was also used in European patent application no. EP11161954.0. and in PCT/IB2011/054628. Metal free organic sensitizers such as DPP based compounds are disclosed, for example, in PCT/IB2013/056648 and in European patent application no. EP12182817.2.

The term "perovskite", for the purpose of this specification, refers to the "perovskite structure" and not specifically to the perovskite material, CaTiO3. For the purpose of this specification, "perovskite" encompasses and preferably relates to any material that has the same type of crystal structure as calcium titanium oxide and of materials in which the bivalent cation is replaced by two separate monovalent cations. The perovskite structure has the general stoichiometry AMX₃, where "A" and "M" are cations and "X" is an anion. The "A" and "M" cations can have a variety of charges and in the original Perovskite mineral (CaTiO₃), the A cation is divalent and the M cation is tetravalent. For the purpose of this invention, the perovskite formulae includes structures having three (3) or four (4) anions, which may be the same or different, and/or one or two (2) organic cations, and/or metal atoms carrying two or three positive charges, in accordance with the formulae presented elsewhere in this specification.

According to an embodiment, the photovoltaic device of the invention comprises one or more layer of an organic-inorganic perovskite. In said device, the last upper layer of organic-inorganic perovskite is coated by the hole transporting layer comprising a hole transporting material as defined above, preferably comprising a compound of formula (I) and/or of formula (II) and/or of formula (III).

According to an embodiment, the sensitizer layer comprises or consists of a nanocomposite material or an organic-inorganic pigments.

According to a further embodiment, the organic-inorganic perovskite layer material comprises a perovskite-structure of any one of formulae (IV), (V), (VI), (VII), (VIII) and (IX) below:

AA'MX₄ (IV)

AMX₃ (V)

AA'N_{2/3}X₄ (VI)

AN_{2/3}X₃ (VII)

BN_{2/3}X₄ (VIII)

BMX₄ (IX)

wherein,
- A and A' are organic, monovalent cations that are independently selected from primary, secondary, tertiary or quaternary organic ammonium compounds, including N-containing heterorings and ring systems, A and A' having independently from 1 to 60 carbons and 1 to 20 heteroatoms;
- B is an organic, bivalent cation selected from primary, secondary, tertiary or quaternary organic ammonium compounds having from 1 to 60 carbons and 2-20 heteroatoms and having two positively charged nitrogen atoms;
- M is a divalent metal cation selected from the group consisting of Cu²⁺, Ni²⁺, Co²⁺, Fe²⁺, Mn²⁺, Cr²⁺, Pd²⁺, Cd²⁺, Ge²⁺, Sn²⁺, Pb²⁺, EU²⁺, or Yb²⁺,
- N is selected from the group of Bi³⁺ and Sb³⁺; and,
- X is independently selected from Cl⁻, Br⁻, I⁻, NCS⁻, CN⁻, and NCO⁻.

In particular, the three or four X may be the same or different. For example, in AMX₃ (formula (V)) may be expressed as formula (V') below:

AMX^{I}XⁱⁱXⁱⁱⁱ (V')

wherein Xⁱ, Xⁱⁱ , Xⁱⁱⁱ are independently selected from Cl⁻, Br⁻, I⁻, NCS⁻, CN⁻, and NCO⁻, preferably from halides (Cl⁻, Br⁻, I⁻), and A and M are as defined elsewhere in this specification. Xⁱ, Xⁱⁱ, Xⁱⁱⁱ may thus be the same or different in this case. The same principle applies to the perovskites of formulae (IV) and (VI)-(IX) and the more specific embodiments of formulae (XI) to (XVII) below. In case of AA'MX₄ (formula IV), for example, formula (IV') applies:

AA'M XⁱXⁱⁱXⁱⁱⁱ X^{iv} (IV')

wherein Xⁱ, Xii , Xⁱⁱⁱ are independently selected from Cl⁻, Br⁻, I⁻, NCS⁻, CN⁻, and NCO⁻, preferably from halides (Cl⁻, Br⁻, I⁻).

Preferably, if Xⁱ, Xⁱⁱ, Xⁱⁱⁱ in formulae (V) and (VII) or Xⁱ, Xii , Xⁱⁱⁱ, X^{iv} in formulae (IV), (VI), (VIII) or (IX) comprise different anions X, there are not more than two different anions. For example, Xⁱ and Xⁱⁱ being the same with Xⁱⁱⁱ being an anion that is different from Xⁱ and Xⁱⁱ.

According to a preferred embodiment, the perovskite material has the structure selected from one or more of formulae (IV) to (VI), preferably (V) or V').

According to a preferred embodiment, said organic-inorganic perovskite layer comprises a perovskite-structure of any one of the formulae (XI) to (XVII):

APbX₃ (XI)

ASnX₃ (XII)

ABix₄ (XIII)

AA'PbX₄ (XIV)

AA'SnX₄ (XV)

BPbX₄ (XVI)

BSnX₄ (XVII)

wherein A, A', B and X are as defined elsewhere in this specification. Preferably, X is preferably selected from Cl⁻, Br⁻ and I⁻, most preferably X is I⁻.

According to a preferred embodiment, said organic-inorganic perovskite layer comprises a perovskite-structure of the formulae (XI) to (XV), more preferably (XI) and/or (XII) above.

According to an embodiment, A and A' are monovalent cations selected independently from any one of the compounds of formulae (37) to (44) below: 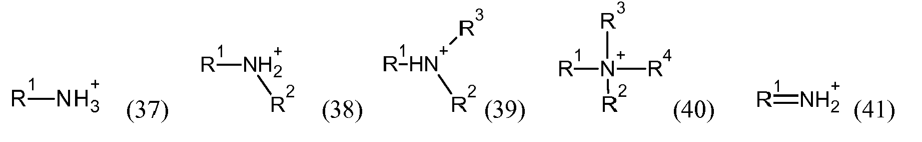 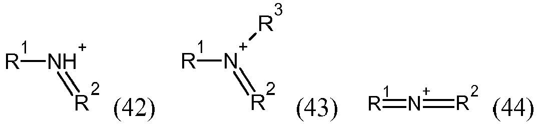 wherein,
any one of R¹, R², R³ and R⁴ is independently selected from C1-C15 organic substituents comprising from 0 to 15 heteroatoms.

According to an embodiment of said C1-C15 organic substituent any one, several or all hydrogens in said substituent may be replaced by halogen and said organic substituent may comprise up to 15 N, S or O heteroatoms, and wherein, in any one of the compounds (37) to (44), the two or more of substituents present (R¹, R², R³ and R⁴, as applicable) may be covalently connected to each other to form a substituted or unsubstituted ring or ring system. Preferably, in a chain of atoms of said C1-C15 organic substituent, any heteroatom is connected to at least one carbon atom. Preferably, neighboring heteroatoms are absent and/or heteroatom-heteroatom bonds are absent in said C1-C15 organic substituent comprising from 0 to 15 heteroatoms. The heteroatoms may be selected from N, S, and/or O.

According to an embodiment any one of R¹, R², R³ and R⁴ is independently selected from C1 to C15 aliphatic and C4 to C15 aromatic or heteroaromatic substituents, wherein any one, several or all hydrogens in said substituent may be replaced by halogen and wherein, in any one of the compounds (37) to (44), the two or more of the substituents present may be covalently connected to each other to form a substituted or unsubstituted ring or ring system.

According to an embodiment, B is a bivalent cation selected from any one of the compounds of formulae (45) and (46) below: 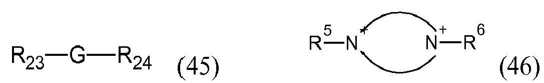 wherein,
in the compound of formula (45), G is an organic linker structure having 1 to 10 carbons and 0 to 5 heteroatoms selected from N, S, and/or O, wherein one or more hydrogen atoms in said G may be replaced by halogen;
wherein any one of R₂₃ and R₂₄ is independently selected from any one of the substituents (47) to (52) below: 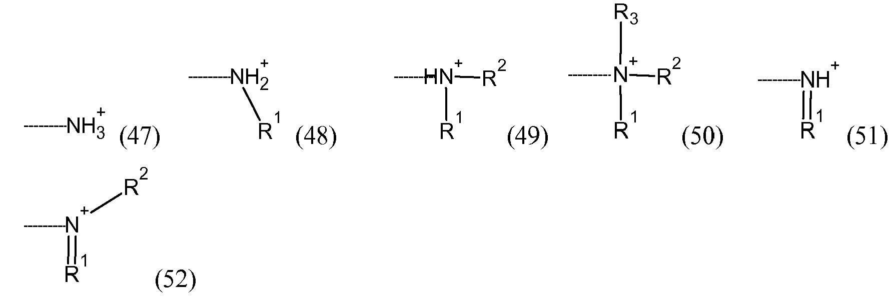
wherein the dotted line in the substituents (47) to (52) represents the bond by which said substituent is connected to the linker structure G;
wherein R¹, R², and R³ are independently as defined above with respect to the compounds of formulae (37) to (44);
wherein R₂₃ and R₂₄, if they are both different from substituent (47), may be covalently connected to each other by way of their substituents R¹, R², and/or R³, as applicable, and wherein any one of R¹, R², and R³, if present, may be covalently connected to G or the ring structure of compound (45), independently from whether said substituent is present on R₂₃ or R₂₄;
and wherein, in the compound of formula (46), the circle containing said two positively charged nitrogen atoms represents a substituted or unsubstituted aromatic ring or ring system comprising 4 to 15 carbon atoms and 2 to 7 heteroatoms or 4 to 10 carbon atoms and 2 to 5 heteroatoms, wherein said nitrogen atoms are ring heteroatoms of said ring or ring system, and wherein the remaining of said heteroatoms may be selected independently from N, O and S and wherein R⁵ and R⁶ are independently selected from H and from substituents as R¹ to R⁴. Halogen atom substituting hydrogen atom totally or partially may also be present in addition to and/or independently of said 2 to 7 heteroatoms.

Preferably, if the number of carbons is in G is impair, the number of heteroatoms is smaller than the number of carbons. Preferably, in the ring structure of formula (46), the number of ring heteroatoms is smaller than the number of carbon atoms. According to an embodiment, G is an aliphatic, aromatic or heteroaromatic linker structure having from 1 to 10 carbons.

Preferably, the dotted line in substituents (47) to (52) represents a carbon-nitrogen bond, connecting the nitrogen atom shown in the substituent to a carbon atom of the linker.

According to an embodiment, in the compound of formula (45), G is an organic linker structure having 1 to 8 carbons and from 0 to 4 N, S and/or O heteroatoms or having 1 to 6 carbons and from 0 to 3 N, S and/or O heteroatoms, wherein any one, several or all hydrogens in said G may be replaced by halogen. Preferably, L is an aliphatic, aromatic or heteroaromatic linker structure having 1 to 8 carbons, wherein any one, several or all hydrogens in said G may be replaced by halogen. According to an embodiment, in the compound of formula (45), said linker G is free of any O or S heteroatoms. According to an embodiment, G is free ofN, O and/or S heteroatoms.

According to an embodiment, any one of R¹, R², R³ and R⁴ is independently selected from C1 to C10 alkyl, C2 to C10 alkenyl, C2 to C10 alkynyl, C4 to C10 heteroaryl and C6 to C10 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R¹-R⁴ may be replaced by halogen.

According to an embodiment, any one of R¹, R², R³ and R⁴ is independently selected from C1 to C8 alkyl, C2 to C8 alkenyl, C2 to C8 alkynyl, C4 to C8 heteroaryl and C6 to C8 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R¹-R⁴ may be replaced by halogen.

According to an embodiment, any one of R¹, R², R³ and R⁴ is independently selected from C1 to C6 alkyl, C2 to C6 alkenyl, C2 to C6 alkynyl, C4 to C6 heteroaryl and C6 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R¹-R⁴ may be replaced by halogen.

According to an embodiment, any one of R¹, R², R³ and R⁴ is independently selected from C1 to C4 alkyl, C2 to C4 alkenyl and C2 to C4 alkynyl, wherein said alkyl, alkenyl and alkynyl, if they comprise 3 or more carbons, may be linear, branched or cyclic, and wherein several or all hydrogens in R¹-R⁴ may be replaced by halogen.

According to an embodiment, any one of R¹, R², R³ and R⁴ is independently selected from C1 to C3, preferably C1 to C2 alkyl, C2 to C3, preferably C2 alkenyl and C2 to C3, preferably C2 alkynyl, wherein said alkyl, alkenyl and alkynyl, if they comprise 3 or more carbons, may be linear, branched or cyclic, and wherein several or all hydrogens in R¹-R⁴ may be replaced by halogen.

According to an embodiment, any one of R¹, R², R³ and R⁴ is independently selected from C1 to C4, more preferably C1 to C3 and even more preferably C1 to C2 alkyl. Most preferably, any one of R¹, R², R³ and R⁴ are methyl. Again, said alkyl may be completely or partially halogenated.

According to an embodiment, A, A' and B are monovalent (A, A') and bivalent (B) cations, respectively, selected from substituted and unsubstituted C5 to C6 rings comprising one, two or more nitrogen heteroatoms, wherein one (for A and A') or two (for B) of said nitrogen atoms is/are positively charged. Substituents of such rings may be selected from halogen and from C1 to C4 alkyl, C2 to C4 alkenyl and C2 to C4 alkynyl as defined above, preferably from C1 to C3 alkyl, C3 alkenyl and C3 alkynyl as defined above. Said ring may comprise further heteroatoms, which may be selected from O, N and S. Bivalent organic cations B comprising two positively charged ring N-atoms are exemplified, for example, by the compound of formula (46) above. Such rings may be aromatic or aliphatic.

A, A' and B may also comprise a ring system comprising two or more rings, at least one of which being from substituted and unsubstituted C5 to C6 ring as defined as above. The elliptically drawn circle in the compound of formulae (46) may also represent a ring system comprising, for example, two or more rings, but preferably two rings. Also if A and/or A' comprises two rings, further ring heteroatoms may be present, which are preferably not charged, for example.

According to an embodiment, however, the organic cations A, A' and B comprise one (for A, A'), two (for B) or more nitrogen atom(s) but are free of any O or S or any other heteroatom, with the exception of halogens, which may substitute one or more hydrogen atoms in cation A and/or B.

A and A' preferably comprise one positively charged nitrogen atom. B preferably comprises two positively charged nitrogen atoms.

A, A' and B may be selected from the exemplary rings or ring systems of formulae (53) and (54) (for A) and from (55) to (57) (for B) below: 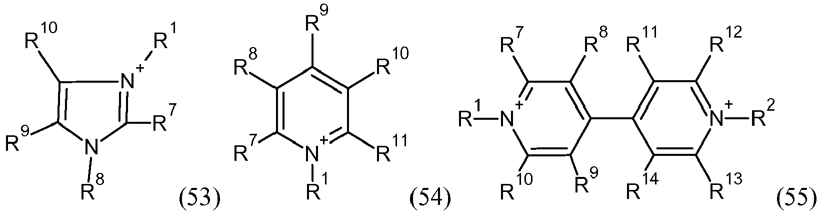 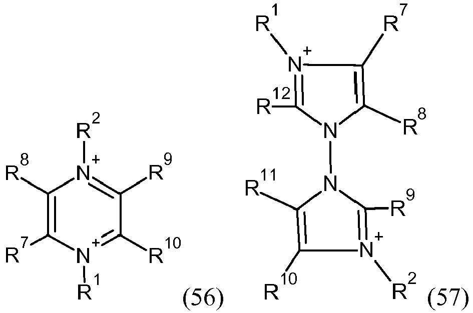 in which R¹ and R² are, independently, as defined above and R⁷, R⁸, R⁹, R¹⁰, R¹¹, R¹², R¹³ and R¹⁴ are independently selected from H, halogen and substituents as defined above for R¹ to R⁴. Preferably, R⁷, R⁸, R⁹, R¹⁰, R¹¹, R¹², R¹³ and R¹⁴ are selected from H and halogen, most preferably H.

In the organic cations A, A' and B, hydrogen atoms may be substituted by halogens, such as F, Cl, I, and Br, preferably F or Cl. Such a substitution is expected to reduce the hygroscopic properties of the perovskite layer or layers and may thus provide a useful option for the purpose of the present specification.

According to a preferred embodiment, A and A' are independently selected from organic cations of formula (37). Preferably, R¹ in the cation of formula (37) is selected from C1 to C8 organic substituents comprising, from 0 to 4 N, S and/or O heteroatom. More preferably, R¹ is selected from C1 to C4, preferably C1 to C3 and most preferably C1 to C2 aliphatic substituents.

According to a preferred embodiment, the metal M is selected from Sn²⁺ and Pb²⁺, preferably Pb²⁺. According to a preferred embodiment, N is Sb³⁺.

According to a preferred embodiment, the three or four X are independently selected from Cl⁻, Br⁻, and I⁻.

According to a preferred embodiment, the organic-inorganic perovskite material has the formula of formulae (XVIII) to (XXII) below:

AMI₃ (XVIII)

AMI₂Br (XIX)

AMI₂Cl (XX)

AMBr₃ (XXI)

AMCl₃ (XXII)

wherein A and M are as defined elsewhere in this specification, including the preferred embodiments of A and M, such as those defined below. Preferably, M is selected from Sn²⁺ and Pb²⁺. Preferably, A is selected from organic cations of formula (37). Preferably, R⁴⁸ in the cation of formula (37) is selected from C1 to C8 organic substituents comprising, from 0 to 4 N, S and/or O heteroatom. More preferably, R¹ is selected from C1 to C4, preferably C1 to C3 and most preferably C1 to C2 aliphatic substituents.

According to a preferred embodiment, the organic-inorganic perovskite is a compound of formula (VII) (AMXiXiiXiii), wherein A is a monovalent cation of formula (37) as defined above, M is Sn²⁺ or Pb²⁺, and Xi, Xii , Xiii are independently selected from Cl⁻, Br⁻, I⁻. Preferably, R¹ in the cation of formula (1) is selected from C1 to C4, preferably C1 to C3 and most preferably C1 to C2 aliphatic substituents. Preferably, Xi -Xiii are identical.

In a further aspect, the invention provides a use of a compound of formula (I) and/or of formula (II) and/or of formula (III) as a hole transporting material in photovoltaic solid state device.

Without to be bound by the theory, said compound of the invention of formula (I) may be used as a light absorbing material to improve the UV-NIR spectral absorption.

The present invention will now be illustrated by way of examples. These examples do not limit the scope of this invention, which is defined by the appended claims.

### Examples

### Example 1: Synthesis of compound of formula (35), namely compound 1 and of compound of formula (36), namely compound 2

The synthetic routes of compounds 1 and 2 are displayed in Figure 1A.

The key building block 3,3',3",4'-tetrabromo-2,2':5',2"-terthiophene 5 was prepared in 75% yield by Pd-catalyzed Negishi coupling of (3-bromothien-2-yl)zinc(II) chloride 4 (2.5 eq.) and tetrabromothiophene 3. Pd-catalyzed tandem Buchwald-Hartwig coupling of 5 with 2-ethylhexyl amine afforded ring-fused S,N-pentacene 6 in 60% yield. Corresponding bis-stannylated derivative 7 was obtained by lithiation of 6 using n-BuLi followed by quenching with trimethyltin chloride. Compounds 1 (formula (35)) and 2 (formula (36)) were finally synthesized in yield of 80% and 82%, respectively, by Pd-catalyzed Stille-type coupling of 7 with dicyanovinylene (DCV)-substituted iodothiophene 8 and iodobithiophene 9. 2-[(3-hexyl-5-iodothiophen-2-yl)methylene]malononitrile 8 was prepared by Knoevenagel condensation of aldehyde 10 and malononitrile according to the following scheme:

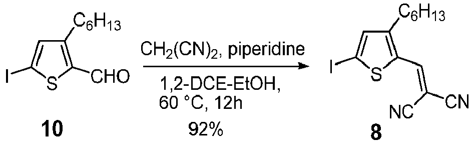

### 2,2'-[(4,5-Bis(2-ethylhexyl)-dithieno[2,3-d:2',3'-d']thieno[3,2-b:4,5-b]dipyrrole-2,7-diyl)bis(3-hexylthien-5,5'-diyl)bis(methane-1-yl-1-ylidine)]dimalononitrile: compound I or of formula (35).

A solution of distannylated derivative 7 (173 mg, 0.21 mmol) and 2-iodothiophene 8 (170 mg, 0.46 mmol) in 5 mL of dry DMF was carefully degassed. Then tetrakis(triphenylphos-phine)-palladium(0) catalyst (14.1 mg, 12.2 µmol) was added and the reaction mixture was stirred at 70°C overnight. After cooling down to room temperature the precipitate was filtered and washed with methanol. The solid was purified by column chromatography (flash-SiO₂, dichloromethane) to afford 1 as a green solid (165 mg, 80%).

Mp 294 °C (DSC); ¹H-NMR (400 MHz, CDCl₃, δ): 7.78 (s, 2H), 7.34 (s, 2H), 7.10 (s, 2H), 4.34-4.21 (m, 4H), 2.74 (t, J= 7.8 Hz, 4H), 2.04-1.94 (m, 2H), 1.69-1.62 (m, 4H), 1.43-1.34 (m, 12H), 1.27-1.16 (m, 16H), 0.93-0.90 (m, 6H), 0.85-0.78 (m, 12H); ¹³C-NMR (100 MHz, CD₂Cl₂, δ): 157.51, 149.41, 146.76, 145.90, 145.87, 132.02, 131.87, 127.92, 124.75, 119.73, 118.79, 118.77, 115.50, 114.44, 111.21, 72.08, 53.44, 40.49, 31.51, 31.26, 30.05, 30.00, 29.25, 29.08, 28.20, 28.13, 23.50, 23.48, 22.96, 22.94, 22.53, 14.05, 13.89, 13.88, 10.62, 10.58; MS (MALDI-TOF) m/z: [M]⁺ calcd for C₅₆H₆₆N₆S₅, 982.40; found, 982.82. HRMS (MALDI-TOF) m/z: [M]⁺ calcd for C₅₆H₆₆N₆S₅, 982.39470; found, 982.39364 [M⁺], 998.38784 [M+O]⁺, 1014.38301 [M+2O]⁺, 1030.37792 [M+3O]⁺. Anal. calcd for C₅₆H₆₆N₆S₅: C 68.39, H 6.76, N 8.55, S 16.30; found: C 68.37, H 6.79, N 8.47, S 16.44.

### 2,2'-[(4,5-Bis(2-ethylhexyl)-dithieno[2,3-d:2',3'-d']thieno[3,2-b:4,5-b]dipyrrole-2,7-diyl)bis(4,3'-dihexyl-2,2'-bithien-5,5'-diyl)bis(methane-1-yl-1-ylidine)]dimalononitrile: compound 2 or of formula (36).

A solution of distannylated derivative 7 (188 mg, 0.23 mmol) and iodo-bithiophene 9 (271 mg, 0.50 mmol) in 5 mL of dry DMF in a schlenk tube was carefully degassed. Then tetrakis(triphenylphosphine)palladium(0) catalyst (16.1 mg, 13.9 µmol) was added and the reaction mixture was stirred at 70°C overnight. After cooling down to room temperature the precipitate was filtered and washed with methanol. The solid was purified by column chromatography (Flash-SiO₂, dichloromethane) to afford 2 as a brown-golden solid (247 mg, 82%).

Mp 275 °C (DSC); ¹H-NMR (400 MHz, CDCl₃, δ): 7.80 (s, 2H), 7.12 (s, 2H), 7.09 (s, 2H), 7.05 (s, 2H), 4.33-4.20 (m, 4H), 2.84 (t, J = 7.9 Hz, 4H), 2.75 (t, J = 7.7 Hz, 4H), 2.06-1.96 (m, 2H), 1.76-1.70 (m, 4H), 1.68-1.62 (m, 4H), 1.49-1.45 (m, 4H), 1.41-1.32 (m, 20H), 1.28-1.18 (m, 16H), 0.93-0.89 (m, 12H), 0.87-0.80 (m, 12H); ¹³C-NMR (100 MHz, CD₂Cl₂, δ): 156.48, 147.22, 147.06, 145.07, 144.91, 144.89, 140.20, 132.56, 130.82, 128.81, 127.11, 126.68, 126.36, 117.55, 117.18, 115.41, 114.11, 108.79, 99.96, 72.94, 53.23, 40.45, 31.65, 31.53, 31.29, 30.39, 30.14, 30.10, 30.08, 30.06, 29.24, 29.13, 29.06, 28.26, 28.17, 23.48, 23.45, 22.99, 22.96, 22.58, 22.54, 14.09, 14.05, 13.93, 13.92, 10.61, 10.56; MS (MALDI-TOF) m/z: [M]⁺ calcd for C₇₆H₉₄N₆S₇,1314.56; found, 1314.66. HRMS (MALDI-TOF) m/z: [M]⁺ calcd for C₇₆H₉₄N₆S₇, 1314.55794; found, 1314.55618 [M⁺], 1330.54740 [M+O]⁺, 1346.54369 [M+2O]⁺, 1362.539 [M+30]⁺. Anal. calcd for C₇₆H₉₄N₆S₇: C 69.36, H 7.20, N 6.39, S 17.06; found: C 69.58, H 7.48, N 6.28, S 16.96.

### Example 2. Optoelectronic characterization of compound of formula (35), namely compound 1 and of compound of formula (36), namely compound 2

The UV-visible light absorption spectra of compounds 1 and 2 in dichloromethane solution are shown in Figures 2A. Compound 1 showed an intensive charge-transfer absorption band at 655 nm with high molar extinction coefficient ε of 117600 Lmol⁻¹cm⁻¹. In contrast, the longer compound 2 comprising bithiophene unit is blue-shifted to 630 nm with an ε value of 86300 Lmol⁻¹cm⁻¹. The appearance of an additional broad and intense band at 430 nm for compound **2** can be assigned to the π-π* transition of the DCV-substituted bithiophene moiety. The absorption band of compounds **1** and **2** coated on TiO₂ films are significantly red-shifted to 725 and 675 nm, respectively. Unlike spiro-MeOTAD and PTAA, which mainly absorbs below 400 nm, both compounds **1** and **2** have strong light harvesting ability in the visible to near-infrared region. To further investigate the contribution of light absorption from the compounds **1** and **2** as HTMs, the UV-Visible spectra of perovskite films on TiO₂ with and without HTM were recorded. As shown in Figure 2B, the perovskite itself has strong absorption between 400-500 nm, and drops dramatically from 600 nm. While both compounds **1** and **2** have the strong absorption band between 600-800 nm, which fully compensates with that of the perovskite. It is quite clear to see that in the presence of HTM, the TiO₂/CH₃NH₃PbI₃/HTM films showed a significant enhancement in the absorption between 550-800 nm, thus, contributing to the light absorption in the low energy region.

In cyclic voltammetry measurement two 1e- oxidation waves were observed for compound 1 and 2, assigned to the formation of stable radical cations and dications, typical for the oligothiophene backbone. The irreversible 2e- reduction wave was assigned to the simultaneous one-electron transfer to the terminal DCV groups. The oxidation potentials for compound 2 were negatively shifted by 190 mV and 300 mV compared to compound 1 due to the increasing electron donating strength by two additional hexylthiophenes. The HOMO and lowest unoccupied molecular orbital (LUMO) energy levels of the compounds 1 and 2 were determined from the onset of the oxidation and reduction waves. These data are graphically shown in Figure 1B. The HOMO energies of compounds 1 and 2 were suitable for their use as HTMs in heterojunction solar cells containing CH₃NH₃PbI₃ perovskite as light harvester and mesoporous TiO₂ as electron transport layer. In comparison to compound 2, the lower HOMO energy level of compound 1 could lead to higher open-circuit voltage (Voc).

As a HTM, the hole mobility of the material has a substantial influence on the effective charge transport in the device. The hole mobilities of pristine molecules were measured in device structure ITO/PEDOT:PSS/oligomer/Al. By using space charge limiting current (SCLC) model, the hole mobilites were estimated to be 0.9 × 10⁻⁴ cm²V⁻¹s⁻¹ for 1 and 0.7 × 10⁻⁴ cm²V⁻¹s⁻¹ for **2.** Besides efficient hole transport ability, the presence of these molecules also contributes to the photocurrent generation. Under illumination, they can be excited together with perovskite, followed by electron transport from the LUMO levels of the molecules to the conduction band of the perovskite. Therefore, photo-excitation in both perovskite and HTM occur together, producing a dual light absorbing system.

For the device preparation, the deposition of CH₃NH₃PbI₃ on mespoporous-TiO₂ film was prepared in two steps, first, by spin-coating of 1.3 M PbI₂ solution in N,N-dimethylformamide (DMF), followed by dip-coating of the TiO₂/PbI₂ film into a solution of CH₃NH₃I in 2-propanol. The dip-coating process resulted in the conversion of CH₃NH₃PbI₃. After annealing of the perovskite film, HTM comprising compound **1** or **2** was subsequently deposited by spin-coating from tetrachloroethane. As seen from the scanning electron microscopy (SEM) cross-section image in Figure 1B, the HTM penetrates into the remaining space of the pores in TiO₂/perovskite layer and at the same time forms a thin capping layer on the top. Finally, the devices were completed by evaporation of a thin gold layer as the back contact.

### Example 3. Photovoltaic characterization of compound of formula (35), namely compound 1 and of compound of formula (36), namely compound 2

Figure 3A shows the current-voltage (*J-V*) characteristics of solar cells based on the structure: FTO/compact TiO₂/mp-TiO₂/CH₃NH₃PbI₃/ compound **1** or compound 2/Au. The reference cell without any HTM was prepared for comparison, which displayed a short-circuit current density (*J*_{SC}) of 13.0 mA cm⁻², *V*_{OC} of 780 mV and fill factor (FF) of 0.69, leading to a PCE of 7.1%. Using compound 1 as HTM the PCE of the devices increased to 10.4%, with a significant increase in both the *J_{SC}* (16.4 mA cm⁻²) and *V*_{OC} (983 mV) values (Table 1). The device with compound **2** as **HTM** generated a *J*_{SC} of 15.2 mA cm⁻², a *V*_{OC} of 886 mV and a FF of 0.68 yielding an overall PCE of 9.3% under standard global AM 1.5 sunlight. The 97 mV lower V_{oc} for compound **2** compared to compound **1** is mainly due to its higher HOMO energy level of compound **2.** Moreover, the absorption band of compound **2** with extended π-conjugation on TiO₂ film is blue-shifted compared to compound **1** and is less intense. Therefore, the light harvesting ability of compound **2** is slightly lower than compound **1** in the wavelength region form 680-770 nm (Figure 2B).

Figure 3B shows the incident-photon-to-current conversion efficiency (IPCE) spectra for the perovskite cells with and without HTMs. The photocurrent generation starts at around 800 nm, in agreement with the bandgap (1.55 eV) of the CH₃NH₃PbI₃, and reaches peak IPCE values of around 76% and 78% for compounds **1** and **2** at 510 nm. The use of compounds **1** and **2** as HTM showed noticeable improvement of the photocurrent in the whole visible region between 400 to 800 nm due to more effective charge extraction. Most importantly, the role of compound **1** as HTM to the photocurrent can be clearly seen between 680-800 nm with IPCE of 48% at 720 nm compared to 27% for the device without HTM, further demonstrating its light harvesting ability together with the perovskite. The excellent agreement of the *J_{SC}* with the value calculated from the overlap integral of the IPCE spectrum with standard AM 1.5G solar emission spectrum showing that the spectral mismatch is negligibly small.

**Table 1: Photovoltaic parameters of the CH₃NH₃PbI₃-based heterojunction solar cells comprising compound 1 or 2 as HTM**

| HTM | J_{SC} [mA cm⁻²] | V_{OC} [mV] | FF | PCE [%] |
|---|---|---|---|---|
| Compound 1 | 16.4 | 983 | 0.64 | 10.4 |
| Compound 2 | 15.2 | 886 | 0.68 | 9.3 |
| No HTM | 13.0 | 780 | 0.69 | 7.1 |

The charge generation in the whole device was further examined by photoinduced absorption spectroscopy (PIA). Figure 4A shows the PIA spectra of the mesoporous TiO₂ films coated by perovskite, by compound **1** as HTM, and by both. For perovskite alone, we observed the features in the near IR region which is assigned to the electrons injected into TiO₂, and a negative band between 700 - 850 nm due to the emission of perovskite itself. TiO₂/compound **1** film without perovskite shows a negative band at wavelength shorter than 870 nm and a positive band beyond 950 nm, due to the ground state bleaching and absorption of the oxidized species (assigned to the hole located on the donor moieties) of compound **1** after photoexcitation. The results indicated that the charge separation occurs between TiO₂ and compound **1** even without perovskite. In the presence of perovskite layer, the absorption features of the oxidized species of compound **1** are more clear obvious, extending from 800 nm to 1400 nm. At the same time, the negative band from the emission of perovskite is quenched. The similar phenomenon was also observed for compound **2.** The results clearly demonstrated the effective charge transfer between photoexcited perovskite and HTM comprising compound 1 or 2, and the formation of long-lived charge species. Therefore high performance devices can be prepared with compounds **1** and **2** as HTMs without using any additives, such as, lithium bis(trifluoromethyl sulfonyl)imide (LiTFSI), 4-tert-butylpyridine (TBP) or even the dopants which were normally used together with spiro-MeOTAD and other semiconducting polymers in order to get higher efficiencies.

In conclusion, two low band gap Acceptor-Donor-Acceptor-type molecules: compounds **1** and **2** comprising electron-rich S,N-heteroacene central units and terminal dicyanovinylene groups were designed and synthesized. The strong red-shifted absorption and appropriate molecular orbital energy levels of these oligomers prompt us to use them as HTM in CH₃NH₃PbI₃-based photovoltaic devices. Solution-processed heterojunction solar cells fabricated with the new HTMs yielded excellent PCEs of 10.4% and 9.3%, respectively which are relatively higher compared to the device without HTM. Besides acting as HTM, they also showed strong light harvesting ability, forming a dual absorbing system together with the perovskite, which opens up new routes for the fabrication and materials selection for perovskite-based photovoltaic devices. Photoinduced absorption spectroscopic studies clearly demonstrate the effective charge transfer between photoexcited perovskite and HTMs. This is the first report on the successful use of A-D-A small molecules as both light harvesting and hole-transporting materials in CH₃NH₃PbI₃-based heterojunction solar cell. We believe that the present findings offer a new avenue to fabricate high-efficiency and low-cost perovskite-based solar cells using colored hole conductors with tunable orbital energy levels, high hole mobility, and additional light harvesting abilities. In addition, due to the high molar extinction coefficient, these molecules can also serves the purpose of light absorber in the long wavelength region where the perovskite absorption is very low.

## Claims

1. A compound of formula (I): 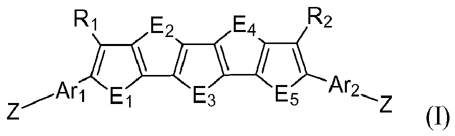 wherein
- E₁, E₂, E₃, E₄ and E₅ are independently selected from the group consisting of O, S, Se, CR₂, SiR₂ or NR, wherein R is selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted;
- R₁ and R₂ are independently selected from H, C1-C20 alkyl, C1-C20 alkoxy, C2-C20 alkenyl, C4-C20 aryl or C1-C20 fluoroalkyl group, wherein said alkyl, alkenyl, alkynyl, alkoxy, and fluoroalkyl, if they comprise 3 or more carbons may be linear, branched or cyclic;
- Ar₁ and Ar₂ are identical or different moieties selected from heteroaromatic ring system having one or more aromatic rings comprising 5 to 40 ring atoms and one or more heteroatoms independently selected from N, S, Se, SiR₂, O or NR, wherein R is defined as above, wherein said aromatic ring may further substituted by one or more moieties independently selected from H, keto (=O) group, fluoro group, C1-C20 alkyl, C1-C20 alkoxy, C2-C20 alkenyl and C2-C20 alkynyl group;
- Z is, on each occurrence, a moiety identically or differently selected from C2-C20 alkenyl, C2-C20 alkynyl, C2-C20 cyanoalkenyl, C2-C20 dicyanoalkenyl, C2-C20 cyanoalkenyl carboxylic ester derivative, C2-C20 alkenyl dicarboxylic ester derivative or C4-C20 heteroaryl, C4-C20 alkenylheteroaryl, wherein one or more heteroatoms are independently selected from O, S or NR, R being defined as above, wherein said alkenyl, cyanoalkenyl, dicyanoalkenyl, cyanoalkenyl carboxylic ester derivative, alkenyl dicarboxylic ester derivative, heteroaryl and alkenyl heteroaryl may be further substituted by one or more moieties independently selected from H, C4-C20 aryl, C1-C20 alkyl, C1-C20 alkoxy group, keto (=O) group, cyano group, C2-C20 dicyanoalkenyl, C2-C20 cyanoalkenyl, carboxylic ester derivative group or dicarboxylic ester derivative group.

2. The compound according to claim 1, wherein E₂ and E₄ are identical moieties selected from the group consisting of O, S, Se, CR₂, SiR₂ and NR and E₁, E₃ and E₅ are identical moieties selected from the group consisting of O, S, Se, O, S, Se, CR₂, SiR₂ and NR, said R being selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted; and wherein said E₂ and E₄ identical moieties are different to said E₁, E₃ and E₅ identical selected moieties.

3. The compound according to claim 1, wherein E₁, E₂, E₄ and E₅ are identical moieties selected from the group consisting of O, S, Se, CR₂, SiR₂ and NR and E₃ is a moiety selected from the group consisting of O, S, Se, CR₂, SiR₂ and NR, wherein R is selected from C1-C20 alkyl, C2-C20 alkenyl, C2-C20 alkynyl, C4-C20 aryl, C2-C20 acetyl and C1-C20 fluoroalkyl group, wherein said alkyl, alkenyl, alkynyl, acetyl and fluoroalkyl, if they comprise 3 or more carbons may be linear, branched or cyclic and wherein aryl group may be substituted; and wherein said E₁, E₂, E₄ and E₅ identical moieties are different to said E₃ selected moiety.

4. The compound according to any one of the preceding claims, wherein Ar₁ and Ar₂ are independently selected from a moiety according to any one of the formulae (1) to (19) 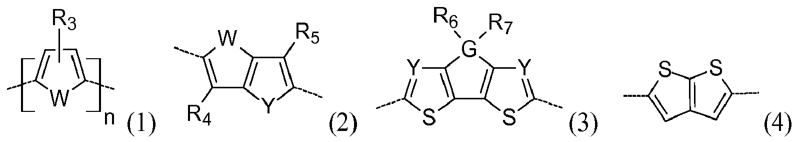 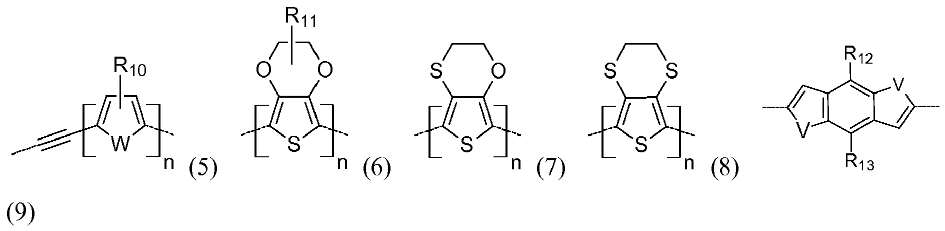 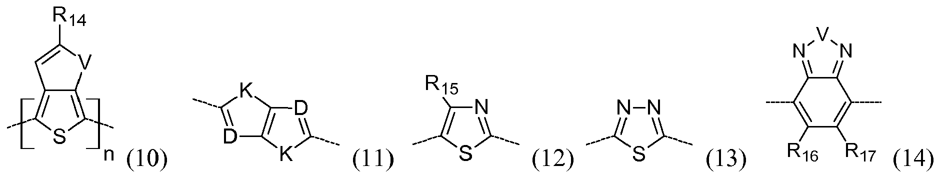 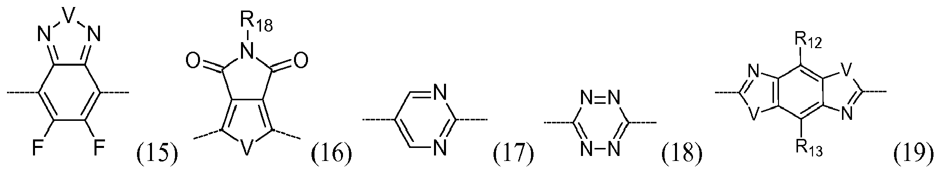 wherein
- W is independently selected from O, S, Se, or NR and Y, V, K and D are independently selected from C, N, S, Se, O, CR₂, SiR₂ or NR, said R being selected from C1-C20 alkyl, C2-C20 alkenyl, C4-C20 aryl and C1-C20 fluoroalkyl group, wherein said alkyl, alkenyl, alkynyl and fluoroalkyl, if they comprise 3 or more carbons may be linear, branched or cyclic and wherein aryl group may be substituted;
- G is selected from C or Si;
- n is an integer from 1 to 10;
- R₃-R₁₈ are independently selected from from H, C1-C20 alkyl, C1-C20 alkoxy, C2-C20 alkenyl.

5. The compound according to claim 4, wherein V is independently selected from O, S or Se and K and D are independently selected form C, O or N.

6. The compound according to any one of the preceding claims, wherein Z is selected from a moiety according to any one of the formulae (20) to (34) and (58) to (66) 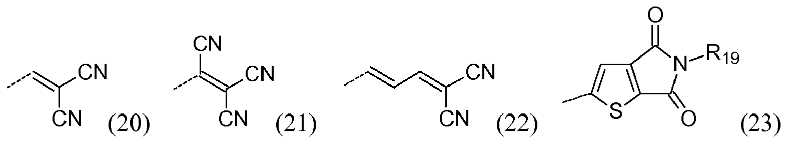 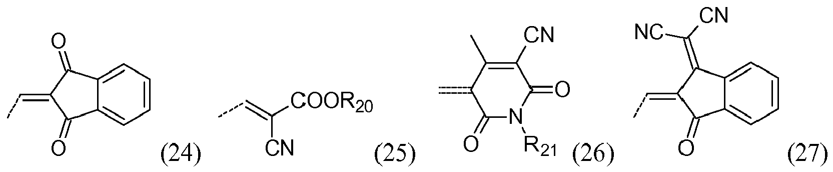 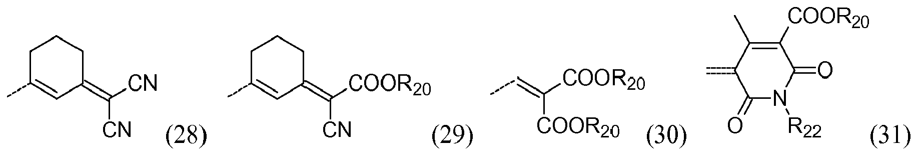 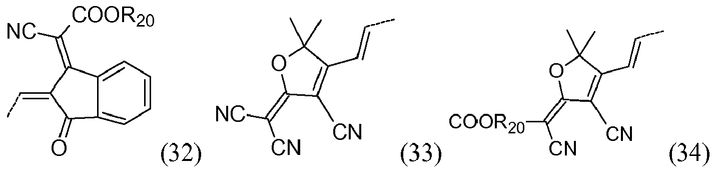 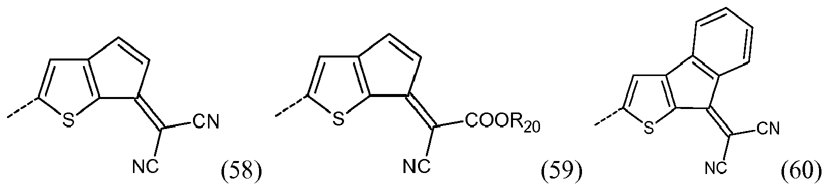 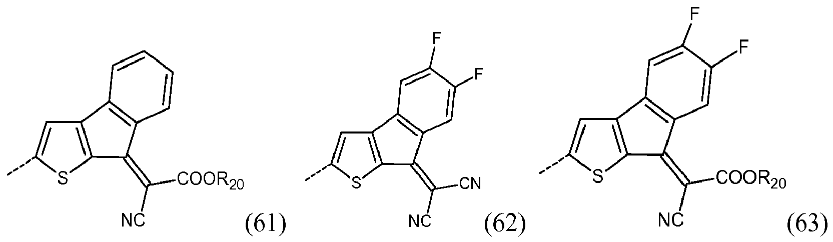 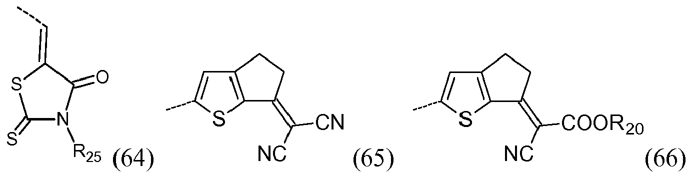 wherein
- COOR₂₀ is a carboxylic ester derivative, wherein R₂₀ is selected from C1-C20 alkyl groups;
- R₁₉, R₂₁, R₂₂ and R₂₅ are independently selected from H, C1-C20 alkyl, C1-C20 alkoxy, C2-C20 alkenyl, C2-C20 alkynyl, C1-C20 fluoroalkyl group and C4-C20 aryl, wherein said alkyl, alkenyl, alkoxy, alkynyl and fluoroalkyl, if they comprise 3 or more carbons may be linear, branched or cyclic and wherein aryl group may be substituted.

7. The compound according to any one of the preceding claims, wherein R₁ and R₂ are identical substituents.

8. The compound according to any one of the preceding claims, wherein Ar₁ and Ar₂ are identical substituents.

9. The compound according to any one of the preceding claims, wherein Z is, on each occurrence, identical substituent.

10. The compound according to any one of the preceding claims, wherein Ar1 and Ar2 are selected from a moiety according to any one of the formulae (1) to (3), (6) to (8), (11) and (12).

11. The compound according to any one of the preceding claims, wherein Z is selected from a moiety according to any one of the formulae (20) to (29), (32) to (34), (58), (60), (62) (64) and (65).

12. A photovoltaic solid state device comprising a compound according to any one of claims 1 to 11.

13. The device according to claim 12 further comprising an organic-inorganic perovskite as sensitizer, said sensitizer being under the form of a layer.

14. The device according to claim 13, wherein the organic-inorganic perovskite layer material comprises a perovskite-structure of any one of formulae (IV), (V), (VI), (VII), (VIII) and (IX) below:
AA'MX₄ (IV)
AMX₃ (V)
AA'N_{2/3}X₄ (VI)
AN_{2/3}X₃ (VII)
BN_{2/3}X₄ (VIII)
BMX₄ (IX)
wherein,
- A and A' are organic, monovalent cations that are independently selected from primary, secondary, tertiary or quaternary organic ammonium compounds, including N-containing heterorings and ring systems, A and A' having independently from 1 to 60 carbons and 1 to 20 heteroatoms;
- B is an organic, bivalent cation selected from primary, secondary, tertiary or quaternary organic ammonium compounds having from 1 to 60 carbons and 2-20 heteroatoms and having two positively charged nitrogen atoms;
- M is a divalent metal cation selected from the group consisting of Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺, or Yb²⁺;
- N is selected from the group of Bi³⁺ and Sb³⁺; and,
- X is independently selected from Cl⁻, Br⁻, I⁻, NCS⁻, CN⁻, and NCO⁻.

15. The device according to any one of claims 12 to 14, wherein said device is selected from a solar cell, a heterojunction, an optoelectronic device and a light emitting device.
